(19) Europäisches Patentamt
European Patent Office
Office européen des brevets

(11) **EP 4 410 964 A1**

(12) **EUROPEAN PATENT APPLICATION**
published in accordance with Art. 153(4) EPC

(43) Date of publication:
**07.08.2024 Bulletin 2024/32**

(21) Application number: **22876600.2**

(22) Date of filing: **19.04.2022**

(51) International Patent Classification (IPC):
*C12N 5/071* (2010.01)    *A61K 35/22* (2015.01)
*A61P 13/12* (2006.01)    *A61K 48/00* (2006.01)

(52) Cooperative Patent Classification (CPC):
**A61K 35/22; A61K 48/00; A61P 13/12; C12N 5/06**

(86) International application number:
**PCT/KR2022/005579**

(87) International publication number:
**WO 2023/054825 (06.04.2023 Gazette 2023/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.09.2021 KR 20210129299**

(71) Applicant: **Ehlbio Co., Ltd.
Uiwang-si, Gyeonggi-do 16006 (KR)**

(72) Inventors:
• **LEE, Hong-Ki
  Seoul 06568 (KR)**
• **LEE, Seong-Hun
  Anyang-si Gyeonggi-do 14038 (KR)**
• **KIM, Sang-Heon
  Incheon 21336 (KR)**
• **JIN, Jeong-Ah
  Seoul 03947 (KR)**
• **KANG, Tae-Wook
  Seoul 08845 (KR)**
• **SO, Hyung-Joon
  Anyang-si Gyeonggi-do 13974 (KR)**
• **SO, Su-Bin
  Incheon 21028 (KR)**
• **JUNG, Kyu-Sung
  Suwon-si, Gyeonggi-do 16498 (KR)**
• **LEE, Jae-Ung
  Siheung-si Gyeonggi-do 14904 (KR)**
• **YOON, Jin-Sang
  Seoul 02407 (KR)**
• **LEE, Young-Jun
  Seoul 05348 (KR)**

(74) Representative: **Murgitroyd & Company
165-169 Scotland Street
Glasgow G5 8PL (GB)**

(54) **MEDIUM COMPOSITION FOR CULTURING URINE-DERIVED STEM CELL, METHOD FOR ISOLATING AND CULTURING URINE-DERIVED STEM CELL USING SAME, URINE-DERIVED STEM CELL WITH IMPROVED THERAPEUTIC FUNCTION FOR RENAL DISEASE, AND CELL THERAPY PRODUCT COMPOSITION CONTAINING SAME**

(57) The present invention relates to a culture medium for isolating and culturing urine-derived stem cells, a method for isolating and culturing urine-derived stem cells using the culture medium, urine-derived stem cells cultured in the culture medium, and a cell therapy product composition containing same.

Fig. 1

## Description

Technical Field

The present disclosure relates to a culture medium for culturing urine-derived stem cells and specifically to a culture medium for enhancing the expression of klotho in urine-derived stem cells. Also, the present disclosure relates to a method for isolating and culturing urine-derived stem cells with improved therapeutic functions for kidney disease.

Background Art

Currently used chronic kidney disease treatments cannot restore damaged kidney tissue, so chronic kidney disease eventually develops into end-stage renal failure. Despite advancements in treatment, the mortality rate remains high (MJ Lee., 2020).

Many efforts have been made to find new treatments to restore the kidney damage, and stem cell therapy has been proposed. Among them, mesenchymal stem cells are recognized for their potential as a new treatment for chronic kidney disease due to their therapeutic efficacy in inflammation and immune response regulation, and angiogenesis. Currently, bone marrow and fat are useful as representative sources of mesenchymal stem cells that can be obtained and differentiated from adults, but since the collection process is invasive, it can cause damage to surrounding tissues. In addition, the low cell yield and purity remain problems to be addressed in applying these cells as a therapy. Therefore, it is necessary to develop a new cell source that can be easily cultured in vitro without causing excessive pain and damage to the patient during the cell collection process and can maintain the characteristics of stem cells well even after long-term culture.

The stem cells of the present disclosure are urine-derived stem cells (UDSC) that can be separated through a simple separation process from human urine and which are derived from renal tubules, renal papillary cells, and epithelial cells of the glomerular wall and are created through the epithelial-mesenchymal transition (EMT) process. It is not yet fully understood how epithelial cells lose cell polarity and change into cells with therapeutic efficacy like mesenchymal stem cells, but it is known that these transformed pluripotent cells play an important role in kidney recovery and regeneration. These urine-derived stem cells can be obtained multiple times without causing any pain or sequelae to the patient, can provide a large number of cells in a short period of time due to their high proliferation rate, and have telomerase activity that is not well expressed in general mesenchymal stem cells, so they are known to have high resistance to cell aging (Qin et al., 2014).

Urine-derived stem cells have the characteristics of self-renewal and therapeutic potential to produce various cells that make up the kidney. In fact, in many studies, urine-derived stem cells can differentiate into various cells experimentally and are known to secrete various paracrine factors that promote angiogenesis. They have also been found to have immune regulatory functions by inhibiting the proliferation of peripheral blood mononuclear cells (PBMC), T cells, B cells, and secreting IL-6 and IL-8 (Qin et al., 2014).

Also, from a regenerative medicine perspective, organ-specific stem cells are generally known to be ideal for treating the organs from which they were separated or originated. For this reason, urine-derived stem cells can be the most suitable option for the treatment and regeneration of the urinary reproductive system and kidneys. Urine-derived stem cells can differentiate into smooth muscle, epithelial and endothelial cells, and podocytes that make up most of the kidney tissue, and these cells are known to lead to the development of kidney-like tissues that can function as kidneys (Qin et al., 2014).

On the other hand, Klotho is known as a representative anti-aging factor. Klotho is a protein discovered through research to extend the lifespan of mice and is known to be mainly expressed in the kidney or pituitary gland (Kuro-o M et al., 1997). Klotho mainly is secreted from the proximal and distal renal tubules in the body and functions like a hormone, is involved in insulin and IGF-1 cell signaling pathways, maintains endocrine homeostasis of calcium, phosphorus, potassium, etc., and is known to function in angiogenesis and antioxidation.

Under normal physiological conditions, the kidney performs an important role in maintaining klotho expression, but it has been revealed in several papers that when the kidney is damaged, the expression of klotho also decreases, and klotho is being studied as an early biomarker for kidney damage (Qi Wang et al., 2018). In addition, many studies have been conducted to observe the recovery from proteinuria and blood sugar and of glomerular filtration rate, etc. by administering recombinant klotho protein to animal models of kidney disease with the klotho gene removed, reporting that klotho is a potential therapeutic factor for chronic kidney damage (Neyra et al., 2017).

According to existing research, Klotho is known to activate recovery mechanisms by enhancing overall cellular metabolic function, such as restoring mitochondrial function in damaged kidneys, maintaining ion channel homeostasis, and enhancing the production of antioxidants. The associated mechanisms are known to involve various signaling pathways such as mTOR, NF-kB, AMPK, Wnt/$\beta$-catenin, Nrf-2, etc. (Marlena T et al., 2021).

As stated in the foregoing, klotho protein is being researched for the development of treatments for various chronic diseases including kidney damage. The methods of delivering klotho protein to patients are mainly mentioned as using small molecules or peptides and using genetic material such as nucleic acids, but the duration of effect and safety are limitations to be overcome. In this regard, the present research team has developed a technology to culture urine-derived stem cells with increased Klotho expression than the conventional culture technology of urine-derived stem cells, and confirmed the efficacy in animal models of kidney disease through the homing effect of urine-derived stem cells (the ability to move to the wound site when administered intravenously) and klotho expression and secretion.

In relation to this, Korean Patent No. 10-2021-0053725 A discloses a medium composition for culturing urine-derived stem cells and a method for culturing urine-derived stem cells using same. However, the document does not disclose at all about a culture solution containing parathyroid hormone (PTH) as an additive.

[Related Art Document]

Patent literature Korean Patent Application No. 10-2019-0139702

Disclosure of Invention

Technical Problem

Through the previous research into characteristics of urine-derived stem cells, the present inventors discovered that urine-derived stem cells retain the therapeutic characteristics of typical mesenchymal stem cells and also express Klotho, a therapeutic factor for kidney disease. This suggests that urine-derived stem cells can be used as a therapeutic agent with higher therapeutic efficacy in kidney-related diseases compared to other stem cells that do not typically express klotho. Many studies have recently been conducted to use klotho as a therapeutic agent in various kidney diseases including chronic kidney disease. From this perspective, urine-derived stem cells, which are stem cells that can naturally secrete klotho, can be an alternative to relatively safe methods for klotho application to humans that do not require special delivery technology. In addition, it was noted that they can be developed into excellent cell therapeutics for kidney disease because of secretion of various therapeutic factors of stem cells.

Leading to the present disclosure, thorough and intensive research conducted by the present inventors resulted in devising a method that can excellently maintain the proliferation rate and stem cell characteristics of urine-derived stem cells even in long-term in vitro culture and can excellently enhance Klotho expression.

An aspect of the present disclosure is to provide a culture medium and a method for isolating and culturing urine-derived stem cells using same, wherein the culture medium is able to excellently maintain the proliferation rate and stem cell characteristics of urine-derived stem cells even in long-term in vitro culture and enhance Klotho expression.

Another aspect of the present disclosure is to provide a method for producing urine-derived stem cells with enhanced kidney disease treatment function through increased klotho expression.

A further aspect of the present disclosure is to provide a cell therapy product that includes, as an active ingredient, urine-derived stem cells with enhanced kidney disease treatment function through increased Klotho expression.

Solution to Problem

A first embodiment of the present disclosure provides a culture medium for the isolation and culture of urine-derived stem cells, wherein the culture medium includes a basic medium containing Dulbecco's Modified Eagle Medium (DMEM) and Ham's F-12, 5% or less fetal bovine serum, and parathyroid hormone (PTH) and may additionally include one or more additives selected from the group consisting of bovine serum albumin, triiodothyronine (T3), and bovine serum albumin.

The basic medium is preferably prepared by mixing of Dulbecco's Modified Eagle Medium (DMEM) and Ham's F-12 at a volume ratio of 1:1 to 5:1, and more preferably at a volume ratio of 2:1 to 3:1.

The fetal bovine serum is preferably added at 0.1% by volume to 10% by volume and more preferably at 2.5 % by volume to 5 % by volume, based on the total volume of the medium.

The bovine serum albumin is preferably contained at a concentration of 0.1 mg/mL to 10 mg/mL based on the total volume of the medium, and more preferably at a concentration of 2.5 mg/mL to 5 mg/mL.

The chemically defined lipids are preferably contained at a concentration of 0.1% by volume to 10% by volume based on the total volume of the medium, and more preferably at a concentration of 1% by volume to 5% by volume.

The FGF23 is preferably included at a concentration of 0.1 ng/mL to 200 ng/mL based on the total volume of the medium, and more preferably at a concentration of 10 ng/mL to 100 ng/mL.

The T3 may be preferably contained in a concentration of 0.1 nM to 40nM, based on the total culture medium, and more preferably in a concentration of 10 nM to 20 nM.

The calcifediol may be preferably contained in a concentration of 1 nM to 1000 nM, based on the total culture medium, and more preferably in a concentration of 100 nM to 500 nM.

The parathyroid hormone (PTH) may be preferably contained in a concentration of 1 nM to 2000 nM, based on the total culture medium, and more preferably in a concentration of 100 nM to 1000 nM.

A second embodiment of the present disclosure provides a method for isolating urine-derived stem cells, the method including the steps of:

    (a) sampling urine from a subject;
    (b) centrifuging the urine to collect cells;
    (c) washing the cells, followed by centrifugation; and
    (d) inoculating the cells with the culture medium.

A third embodiment of the present disclosure provides a method for culturing urine-derived stem cells, the method including a step of inoculating the urine-derived stem cells with the culture medium and culturing same.

The cultured urine-derived stem cells may have:

    (1) an increased cell proliferation rate compared to urine-derived stem cells cultured in KSFM and DMEM/F12 (1:1 volume ratio) medium, and
    (2) increased expression of the stem cell-related genes OCT4, SOX2, and Nanog, the anti-aging-related genes FOXO1 and FOXO3, and the kidney-specific therapeutic factor klotho compared to urine-derived stem cells cultured in KSFM and DMEM/F12 (1:1 volume ratio) medium.

A fourth embodiment of the present disclosure provides urine-derived stem cells cultured in the culture medium.

The urine-derived stem cells may have an enhanced kidney disease treatment function.

The urine-derived stem cells may be positive for CD73, CD90, CD105, CD146, or SSEA-4.

The urine-derived stem cells may be autologous, allogeneic, or xenogeneic.

The urine-derived stem cells may decrease the neutrophil gelatinase-associated lipocalin (NGAL) level.

The kidney disease may be one or more selected from a group consisting of diabetic nephropathy, chronic renal failure, acute renal failure, subacute renal failure, glomerulonephritis, malignant nephrosclerosis, vascular microangiopathy, transplant rejection, glomerulopathy, kidney hypertrophy, kidney proliferation, proteinuria, contrast-induced nephropathy, toxin-induced kidney damage, oxygen free-radical-mediated kidney disease, and nephritis.

A fifth embodiment of the present disclosure provides a cell therapy product with enhanced kidney disease treatment function, wherein the cell therapy product includes urine-derived stem cells as an active ingredient.

The cell therapy product may be a preparation adapted to be administered via an oral, rectal, intravenous (i.v), intraarterial, intraperitoneal, intramuscular, intrasternal, transdermal, local, intraocular, subcutaneous, or intradermal route.

The cell therapy product may further include a pharmaceutical carrier.

Advantageous Effects of Invention

The urine-derived stem cells according to the present disclosure showed effects of improving kidney function and regenerating tissues in a kidney disease animal model. Furthermore, urine-derived stem cells can be cultured at a mass scale and developed into stem cell therapeutics targeting diseases of the urinary system, with the expectation of a use as safe and efficient therapeutics for various intractable diseases.

Brief Description of Drawings

FIG. 1 is a scheme illustrating the isolation and culturing process of urine-derived stem cells.
FIG. 2 shows plots of cell proliferation rates (CPDL and DT) compared and analyzed among urine-derived stem cells cultured in media of Table 3.
FIG. 3 is a table in which cellular characteristics of urine-derived stem cells cultured in media of Table 3 are compared and analyzed.
FIG. 4 shows graphs of expression levels of main genes in urine-derived stem cells as measured by real-time PCR analysis.
FIGS. 5 and 6 show optimal concentrations determined by examining cell proliferation rates or klotho gene expression levels in media different in the concentration of the additive of Table 1.
FIG. 7 shows klotho gene and protein expression levels compared and analyzed in urine-derived stem cells cultured in the media of Table 3.

FIG. 8 shows inhibitory effects of urine-derived stem cells cultured in media of Table 1 on renal fibrosis and podocyte apoptosis as assayed in vitro.

FIG. 9 shows results of examining whether when administered to mouse models of ischemia-reperfusion kidney injury, the urine-derived stem cells cultured in the media of Table 1 moved to the kidney (Homing effect).

FIG. 10 shows results of examining whether when administered to mouse models of ischemia-reperfusion kidney injury, the urine-derived stem cells cultured in the media of Table 1 exhibited therapeutic efficacy.

Best Mode for Carrying out the Invention

The term "stem cells," as used herein, refers to cells that have the potential to differentiate into cells of all tissues in an organism, possessing pluripotency or totipotency, and also exhibit self-renewal capability. The term is intended to encompass embryonic stem cells, induced pluripotent stem cells, and adult stem cells.

As used herein, the term "proliferation" of stem cells refers to the process where the stem cells divide, maintaining their stem cell characteristics without differentiating into specific cells, and thus increasing the total number of cells.

As used herein, the term "culture medium" refers to a composition that contains nutrients necessary for maintaining the growth and survival of cells in vitro.

As used herein, the term "passage culture" refers to a method of continuously culturing cells for a long period of time in a healthy state by periodically transferring a portion of the cells to a new culture vessel and exchanging the culture medium with a fresh one. As the number of cells increases in a culture vessel with limited space, the proliferative nutrients are consumed or contaminants accumulate over time, causing the cells to naturally die. Therefore, it is used as a means to increase the number of healthy cells. Typically, one passage means one round of replacing the culture medium (or culture vessel) once with a fresh one or dividing a population of cells into sub-groups.

As used herein, the term "subject" refers to a target that requires treatment for kidney disease, specifically including mammals such as humans or non-human primates, mice, dogs, cats, horses, and cows.

The term "cell therapy product", as used herein, refers to cells and tissues that have been isolated, cultured, and prepared from a subject through special manipulation and are used as pharmaceuticals for the purpose of treatment, diagnosis, and prevention. It means a pharmaceutical for use in treating, diagnosing, and preventing diseases through a series of actions including proliferating and selecting living autologous cells, allogeneic cells, or xenogeneic cells in vitro or changing the biological characteristics of cells in other ways for the purpose of restoring the function of cells or tissues.

According to a first embodiment thereof, the present disclosure provides a culture medium for the isolation and culture of urine-derived stem cells, wherein the culture medium includes: a basic medium containing Dulbecco's Modified Eagle Medium (DMEM) and Ham's F-12; fetal bovine serum; and parathyroid hormone (PTH) and may further include one or more additives selected from the group consisting of calcifediol, bovine serum albumin, and chemically defined lipids.

According to a second embodiment thereof, the present disclosure provides a method for isolating urine-derived stem cells, the method including the steps of: (a) sampling urine from a subject; (b) centrifuging the urine to collect cells; (c) washing the cells, followed by centrifugation; and (d) inoculating the cells with the culture medium according to claim 1.

The method may additionally include a step of (e) harvesting urine-derived stem cells by treating the cells with trypsin.

In the method, the centrifugation may be performed at 300g to 700g for 5 to 15 minutes, but with no limitations thereto.

In the method, the washing may be performed by PBS (phosphate buffered saline), but with no limitations thereto.

According to a third embodiment thereof, the present disclosure provides a method for culturing urine-derived stem cells, the method including a step of inoculating the urine-derived stem cells with the culture medium and culturing same.

The method may further include step (h) of detaching the cultured urine-derived stem cells at a confluency of 70% to 90% by trypsinization, inoculating the cells at a density of $1 \times 10^3$ to $1 \times 10^4$ (cells/cm$^2$) per area into a larger culture vessel, and then culturing the cells for 5 to 8 passages.

In the method, the washing may be performed by PBS (phosphate buffered saline), but with no limitations thereto.

According to a fourth embodiment thereof, the present disclosure provides urine-derived stem cells cultured in the culture medium.

According to a fifth embodiment thereof, the prevent disclosure provides a cell therapy product with enhanced kidney disease treatment function, wherein the cell therapy product includes urine-derived stem cells as an active ingredient.

In the cell therapy product, the urine-derived stem cells may be autologous, allogeneic, or xenogeneic.

In the cell therapy product, the kidney disease may be one or more selected from a group consisting of diabetic nephropathy, chronic renal failure, acute renal failure, subacute renal failure, glomerulonephritis, malignant nephrosclerosis, vascular microangiopathy, transplant rejection, glomerulopathy, kidney hypertrophy, kidney proliferation, proteinuria, contrast-induced nephropathy, toxin-induced kidney damage, oxygen free-radical-mediated kidney disease, and nephritis.

The cell therapy product may include additional pharmaceutical excipients commonly used in cell therapy. As used herein, the term "pharmaceutically acceptable" means physiologically acceptable so as not to cause allergic reactions or similar reactions such as gastrointestinal disorders, dizziness, and the like, upon administration to humans.

Examples of pharmaceutically acceptable excipients for parenteral administration include, but are not limited to, water, suitable oils, saline, aqueous glucose, and glycol. Stabilizers and preservatives may further be included. Suitable stabilizers may include antioxidants such as sodium bisulfite, sodium metabisulfite, or ascorbic acid. Suitable preservatives may include benzalkonium chloride, methyl- or propyl-parabens, and chlorobutanol. With respect to other pharmaceutically acceptable excipients, reference may be made to the literature (Remington's Pharmaceutical Sciences, 19th ed., Mack Publishing Company, Easton, PA, 1995).

The cell therapy product may also be administered by means of any suitable device that guides the cell therapy product to target cells.

Mode for invention

A better understanding of the present disclosure may be obtained through the following examples which are set forth to illustrate, but are not to be construed as limiting the present disclosure.

EXAMPLE 1: Isolation and Culture of Urine-Derived Stem Cells

A total of 100 ml of urine was filtered through a 100 um filter to remove particulate matter such as mucus. The filtered urine was divided into 50 ml tubes and centrifuged at 500xg for 15 minutes. After centrifugation, the supernatant was discarded, and the cell pellet was collected and washed with 1X PBS (Gibco). The washed cells were centrifuged at 500xg for 10 minutes. This washing process was repeated twice. Then, the supernatant was discarded, and the cell pellet was resuspended in a culture medium with the composition listed in Table 1. The resuspended cells were plated at a density of 500 $\mu$l per well into 24-well plates. The cells were cultured for 5 days at 37°C under 5% $CO_2$ in a humidified incubator to allow the formation of urine-derived stem cell colonies.

TABLE 1

| Basal media | Supplement | Manufacturer | Concentration |
|---|---|---|---|
| DMEM + F12 (3:1 mixed ) | FBS | Gibco | 2.5% |
| | Bovine serum albumin | Sigma-Aldrich | 2.5 mg/ml |
| | Lipid mixture | Gibco | See Table 2 |
| | FGF23 | Sigma-Aldrich | 10 ng/ml |
| | Triiodothyronine(T3 ) | Sigma-Aldrich | 10 nM |
| | Calcifediol | Sigma-Aldrich | 100 nM |
| | PTH | Sigma-Aldrich | 100 nM |
| | Y27632 (added only upon isolation) | STEMCELL Technologies | 10 $\mu$M |

TABLE 2

| Lipid Mixture | Concentration (1% added based on total volume of medium) |
|---|---|
| Arachidonic acid | 20 ng/ml |
| Cholesterol | 2200 ng/ml |
| DL-alpha-Tocopherol Acetate | 700 ng/ml |
| Linoleic acid | 100 ng/ml |
| Myristic acid | 100 ng/ml |

(continued)

| Lipid Mixture | Concentration (1% added based on total volume of medium) |
|---|---|
| Oleic acid | 100 ng/ml |
| Palmitic acid | 100 ng/ml |
| Palmitoleic acid | 100 ng/ml |
| Pluronic F-68 | 900 $\mu$g/ml |
| Stearic acid | 100 ng/ml |
| Tween 80 | 22 $\mu$g/ml |

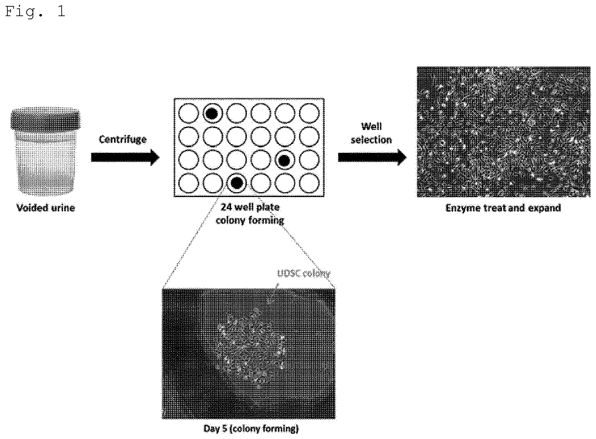

Since the plating, circular cell colonies consisting of tightly packed cells appeared in specific wells of the 24-well plates (FIG. 1). After colony formation, the medium was completely removed and the cells were washed with 1X PBS. The medium with the composition listed in Table 1 was employed in replace thereof. The cells were further cultured for an additional 5 days. The medium was exchanged every 2-3 days with a fresh one. After 10 days of incubation from the initial plating, when the diameter of the urine-derived stem cell colonies increased to approximately 5-10 mm, enzymatic treatment was performed for the first passage. The medium was removed, and the cells were washed with 1X PBS. TrypLE Express (Gibco) was added to each well at a volume of 200 $\mu$l per well and incubated at 37°C in a $CO_2$ incubator for 5 minutes. The enzyme was neutralized with medium containing FBS (Gibco), and the detached cells were collected in 15 ml tubes and centrifuged at 1500 rpm for 5 minutes to obtain a cell pellet. After discarding the supernatant, the cell pellet was resuspended in the second culture medium, and the cells were counted. Subsequently, the cells were inoculated into a larger culture vessel at a concentration of $5\times10^3$ cells per unit area ($cm^2$), and cultured at 37°C, under 5% $CO_2$ in a humidified incubator. The medium was changed every 2-3 days, and when the cell confluence reached 80-90%, the cells were passaged at a concentration of $5\times10^3$ cells per $cm^2$.

Immediately after isolation, the cells contained in the urine included renal tubular cells, uroepithelial cells, squamous epithelial cells, leukocytes, and erythrocytes. Among the cells derived from urine, 99% were non-adherent cells in the cell culture vessel and were removed during medium changes. Of the remaining cells, 0.1% was accounted for by cells derived from uroepithelial cells, muscle cells, and endothelial cells while 0.2% exhibited characteristics of urine-derived stem cells with multipotent differentiation capacity. In this isolation method, non-urine-derived stem cells were eliminated due to differences in proliferation rate compared to urine-derived stem cells. The urine-derived stem cell isolation and culture process are depicted in FIG. 1.

EXAMPLE 2: Cell Proliferation Rate Analysis

For comparison and evaluation of cell proliferation rates, urine-derived stem cells were isolated and cultured using two types of media previously used in prior art (hereinafter, referred to as Media 1, Media 2), the culture medium according to Korean Patent No. 10-2021-0053725 A (hereinafter referred to as TM5-EXP), and the culture medium of the present disclosure (hereinafter referred to as TM8), as given in Table 3.

TABLE 3

| Media 1 | Media 2 | TM5-EXP | TM8 |
|---|---|---|---|
| KSFM+DMEM/F12 | DMEM | | |
| 2.5% FBS | 2.5% FBS | DMEM/F-12 | DMEM/F-12 |
| | EGF (10 ng.ml) | 2.5% FBS | 2.5% FBS |
| BPE (50 $\mu$g/ml) | PDGF (2 ng/ml) | | |
| EGF (10 ng/ml) | TGF-$\beta$ (1 ng/ml) | BSA | BSA |
| Hydrocortisone (0.5 mM) | bFGF (2 mg/ml) | Triiodo-L-thyronine (T3) | Triiodo-L-thyronine (T3) |
| | Hydrocortisone (0.5 mM) | Lipid mixture (CDLC) | Lipid mixture (CDLC) |
| Insulin (25 mg/ml) | | | |
| Transferrin (20 mg/ml) | Insulin (25 mg/ml) | | |
| triiodo-L-thyronine (T3) (50 ng/ml) | Transferrin (20 mg/ml) | FGF23 | FGF23 |
| | | Calcifediol | Calcifediol |
| | adrenaline (549 ng/ml) | | PTH |

(continued)

| Media 1 | Media 2 | TM5-EXP | TM8 |
|---|---|---|---|
| Adenine (0.18 mM) | Triiodo-L-thyronine (T3) (50 ng/ml) | See Table 1 | See Table 1 |

*The cells were passaged every 4 days, and counted for each passage up to the 5th passage. The cell counts were measured using the ADAM cell counter (NanoEnTek, Korea). The cell proliferation rate was evaluated using the following formulas to calculate CPDL (Cumulative Population Doubling Level) and DT (Doubling Time):*

$$PDL = ln(Nf/Ni)/ln2 \; (*CPDL = cumulative \; PDL)$$

$$DT \; (hr) = X*Log2/(Log(Nf)-Log(Ni))*24$$

*(Ni = initial cell count at seeding, Nf = final cell count, X = number of culture days)*

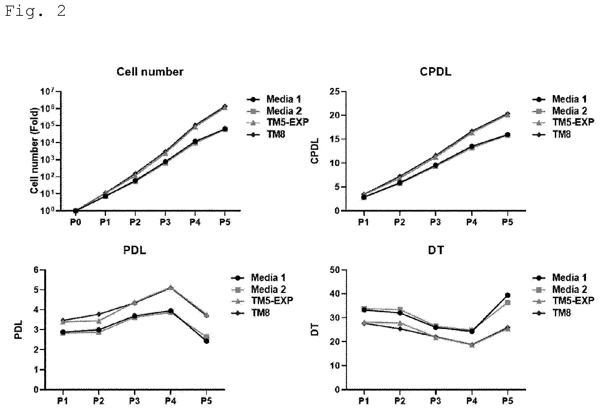

As shown in FIG. 2, urine-derived stem cells cultured with TM5-EXP and TM8 exhibited higher cell proliferation rates compared to those cultured with Media 1 and Media 2.

The analysis of doubling time (DT), which represents the time taken for cells to divide once, at the 5th passage revealed that Media 1 and Media 2 had DT values of 39.26 and 36.38 hours, respectively, while TM5-EXP and TM8 had DT values of 24.44 and 23.99 hours, respectively.

The analysis of cumulative population doubling level (CPDL), which represents the number of cell divisions up to the 5th passage, showed that Media 1 and Media 2 had CPDL values of 15.96 and 15.85, respectively, while TM5-EXP and TM8 had CPDL values of 20.13 and 20.39, respectively.

Based on the cell proliferation rate analysis, urine-derived stem cells cultured with TM5-EXP and TM8 exhibited relatively higher cell proliferation rates and maintained a high cell proliferation rate up to the 5th passage compared to those cultured with Media 1 and Media 2. However, there was no significant difference in the proliferation rate between TM5-EXP and TM8.

EXAMPLE 3: Cell Characterization Analysis

In this Example, urine-derived stem cells cultured with the culture media listed in Table 3 were compared and evaluated for their cell characteristics. The expression of specific surface markers on urine-derived stem cells was analyzed using flow cytometry at the 3rd and 5th passages of cell culture. It is known from previous studies that urine-derived stem cells possess at least one of the markers CD73, CD90, CD105, CD146, SSEA-4 and do not express at least one of CD31, CD34, and CD45. Therefore, analysis focus was imparted to these markers.

Specifically, urine-derived stem cells cultured with the culture media listed in Table 3 were collected at the 3rd and 5th passages, and $1 \times 10^6$ cells were resuspended in 500 $\mu$l of PBS (Gibco). The resuspended cells were distributed into FACS tubes (5 ml, poly styrene roundbottom, Falcon) at 100 $\mu$l per tube. FACS antibodies (Table 6) were added to each tube, and the tubes were incubated in the dark at room temperature for 20 minutes. After incubation, 500 $\mu$l of PBS was added to each tube, and the cells were analyzed using a flow cytometer. The FACSverse™ instrument from BD was used for cell analysis, following the instrument's measurement manual, and the analysis was performed using the analysis software.

TABLE 4

| | Cell Surface Marker | Volume |
|---|---|---|
| CD31 | FITC Mouse Anti-Human CD31 | 5$\mu$l |
| CD34 | PE-Cy5 Mouse Anti-Human CD34 | 5$\mu$l |
| CD45 | FITC Mouse Anti-Human CD45 | 5$\mu$l |
| CD73 | FITC Mouse Anti-Human CD73 | 5$\mu$l |
| CD90 | PE-Cy5 Mouse Anti-Human CD90 | 5$\mu$l |
| CD105 | PE-Cy7 Mouse Anti-Human CD105 | 5$\mu$l |
| CD146 | PE Mouse Anti-Human CD146 | 5$\mu$l |

(continued)

|  | Cell Surface Marker | Volume |
|---|---|---|
| SSEA4 | FITC Mouse Anti-SSEA-4 | 5μl |

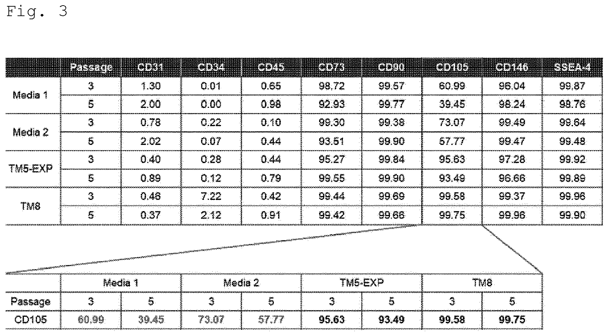

The results are depicted in FIG. 3. As can be seen in FIG. 3, urine-derived stem cells cultured with the culture media listed in Table 3 exhibited positive expression (over 90%) for CD73, CD90, CD146, and SSEA-4 while they showed negative expression (less than 10%) for CD31, CD34, and CD45 at both the 3rd and 5th passages of cell culture. However, there were differences in the expression of CD105, which is closely related to the differentiation characteristics of stem cells, among the different culture media. Urine-derived stem cells cultured with Media 1 and Media 2 showed an average expression rate of 50.22% and 65.42% for CD105, respectively, while those cultured with TM5-EXP and TM8 showed superior expression rates of 94.56% and 99.67%, respectively. When comparing the expression rates among different passages, the Media 1 and Media 2 groups showed a decreasing trend in CD105 expression as the passage proceeded from the 3rd to the 5th round while the TM5-EXP and TM8 groups maintained a relatively constant expression without significant changes. There was no significant difference observed between TM5-EXP and TM8.

The inventors speculated that these differences may be attributed to the various types and concentrations of additives such as extracts from the choroid plexus, growth factors, and hormones added to Media 1 and Media 2, which may have influenced the cell characteristics during long-term culture. On the other hand, the culture medium of the present disclosure included key growth factors and hormones that closely interact with human kidney cells, such as FGF23, Calcifediol, and PTH, while minimizing the addition of unnecessary growth factors and hormones. This may explain the observed differences in cell characteristics.

Therefore, in Example 4, the major gene expression levels of urine-derived stem cells in response to the addition of FGF23, Calcifediol, PTH, and other substances were examined using PCR analysis.

EXAMPLE 4: Gene Expression Analysis

In this Example, real-time polymerase chain reaction (PCR) analysis was conducted to compare and evaluate the gene expression characteristics of urine-derived stem cells cultured with the culture media listed in Table 3 up to the 5th passage.

Urine-derived stem cells from the 5th passage were collected, and $1\times10^6$ cells were centrifuged at 1500 rpm for 5 minutes. The supernatant was discarded and the cells were washed with 1X PBS. The cell pellet, after removing the supernatant, was used for total RNA extraction using the TAKARA MiniBEST Universal RNA Extraction Kit (Cat. #9767). The extracted RNA was then used to synthesize cDNA using the PrimeScript™ 1st strand cDNA Synthesis Kit (Cat. 6110A). The gene expression levels were analyzed using real-time PCR with SYBR Green Premix Ex Tag II (Cat. #RR820S/A/B). The housekeeping gene GAPDH was used as a reference gene, and the expression of stem cell-related genes, such as Oct4, Sox2, and Nanog, was examined. Additionally, the expression of the known senescence-associated genes Foxo1 and Foxo3A was also analyzed. (Table 5)

TABLE 5

|  | Primer | Sequence (5'- 3') |
|---|---|---|
| Housekeeping gene | Gapdh | F - AGC CAC ATC GCT CAG ACA C |
|  |  | R - GCC CAA TAC GAC CAA ATC C |
| Stem cell markers | Oct4 | F - TTC AGC CAA ACG ACC ATC TG |
|  |  | R - CAC GAG GGT TTC TGC TTT GC |
|  | Sox2 | F - CCC ACC TAC AGC ATG TCC TAC TC |
|  |  | R - TGG AGT GGG AGG AAG AGG TAA C |
|  | Nanog | F - TTC CCT CCT CCA TGG ATC TG |
|  |  | R - TGT TTC TTG ACT GGG ACC TTG TC |

(continued)

| | Primer | Sequence (5'- 3') |
|---|---|---|
| Anti-aging markers | Foxo1 | F – TTA TGA CCG AAC AGG ATG ATC TTG |
| | | R – TGT TGG TGA TGA GAG AAG GTT GAG |
| | Foxo3a | F – GCG TGC CCT ACT TCA AGG ATA AG |
| | | R - GAC CCG CAT GAA TCG ACT ATG |
| | Klotho | F - ACA GAG GTT ACA GCA TCA GGC G |
| | | R - TTC CTC TTC TTG GTC ACA ACC |

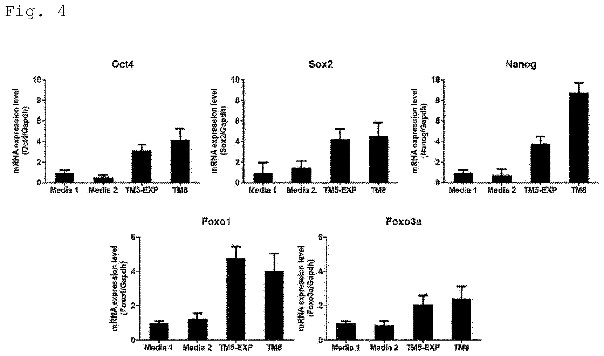

As shown in FIG. 4, urine-derived stem cells cultured with TM5-EXP and TM8 exhibited significantly enhanced expression of the stem cell-related genes Oct4, Sox2, and Nanog, compared to those cultured with Media 1 and Media 2. Particularly, the expression of the Nanog gene was remarkably increased by more than 8-fold. Nanog is a key gene involved in the self-renewal and pluripotency of human embryonic stem cells. The substantial improvement in Nanog expression by TM8 indicated a significant enhancement in the proliferative capacity and differentiation potential of urine-derived stem cells. Additionally, the expression of the known senescence-associated genes Foxo1 and Foxo3a was enhanced in the TM5-EXP and TM8 groups compared to the Media 1 and Media 2 groups. Foxo proteins are known as critical transcription factors involved in cellular senescence and play a protective role against various external stresses. The increased expression of Foxo genes is speculated to confer resistance to cell senescence that may occur during long-term cultivation. This finding is expected to be associated with the previously observed cell proliferation results.

Through these data, it is confirmed that the cultivation of urine-derived stem cells with TM5-EXP and TM8 can enhance stem cell characteristics and cellular anti-aging properties. Particularly, TM8 cultivation significantly improves the expression of genes related to self-renewal and multipotency in urine-derived stem cells.

EXAMPLE 5: Exploration of Optimal Concentrations of Additives in Culture Medium

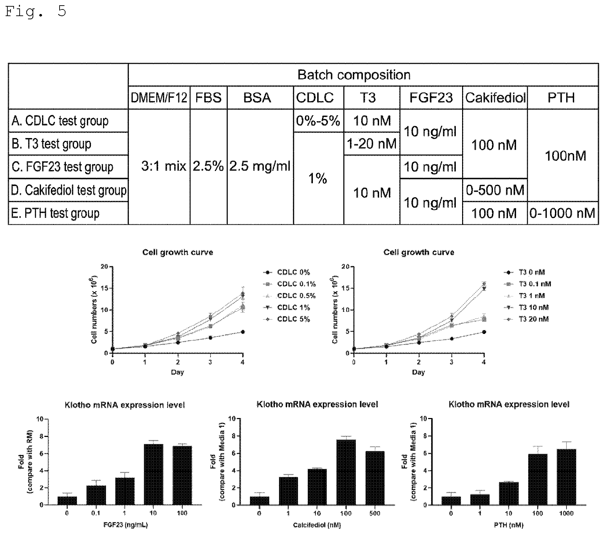

In this Example, cell proliferation rate or klotho gene analysis was conducted with various concentrations of lipid mixture (CDLC), T3, FGF23, Calcifediol, and PTH in the TM8 culture medium to explore their optimal concentrations (see FIG. 5).

Specifically, CDLC was used to compensate for the decrease in cell proliferation rate caused by low serum usage, while T3 was used to facilitate the proliferation of urine-derived stem cells with characteristics of epithelial cells. In this example, evaluation was made of the improvement of cell proliferation rate in urine-derived stem cells in various concentration ranges of lipid mixture and T3 to determine their optimal concentrations.

Furthermore, FGF23, Calcifediol, and PTH that are added to the culture medium of the present disclosure are growth factors and hormones that interact through the endocrine system in the human kidney. The klotho proteins in the human body mediate these interactions. The inventors aimed to enhance the expression of klotho proteins in urine-derived stem cells by mimicking the endocrine system in the human body through the composition of the culture medium. In this Example, evaluation was made of the enhancement of klotho gene expression in urine-derived stem cells in various concentration ranges of FGF23, Calcifediol, and PTH to determine their optimal concentrations.

The results are depicted in FIG. 5. As shown, the addition of CDLC or T3 to the TM8 medium resulted in enhanced cell proliferation compared to the control group, and this effect was dose-dependent. Particularly, the most significant improvement in cell proliferation was observed at CDLC concentrations of 1% to 5% and T3 concentrations of 10 nM to 20 nM, although there were no significant differences among the different concentrations. Therefore, the optimal concentration of CDLC was determined to be 1%, and the optimal concentration of T3 was determined to be 10 nM, considering the enhancement in proliferation relative to the added dosage.

Moreover, the expression of klotho gene in urine-derived stem cells was compared by adding different concentrations of FGF23, Calcifediol, and PTH to the TM8 medium. As shown in FIG. 5, the addition of FGF23, Calcifediol, or PTH resulted in increased klotho gene expression compared to the control group, and this effect was dose-dependent.

The most significant enhancement in klotho gene expression was observed at FGF23 concentrations of 10 ng/mL to 100 ng/mL, Calcifediol concentrations of 100 nM to 500 nM, and PTH concentrations of 100 nM to 1000 nM, although there were no significant differences among the different concentrations. Therefore, the optimal concentration was determined to be 10 ng/mL for FGF23, 100 nM for Calcifediol, and 100 nM for PTH, considering the enhancement in gene expression relative to the added dosage.

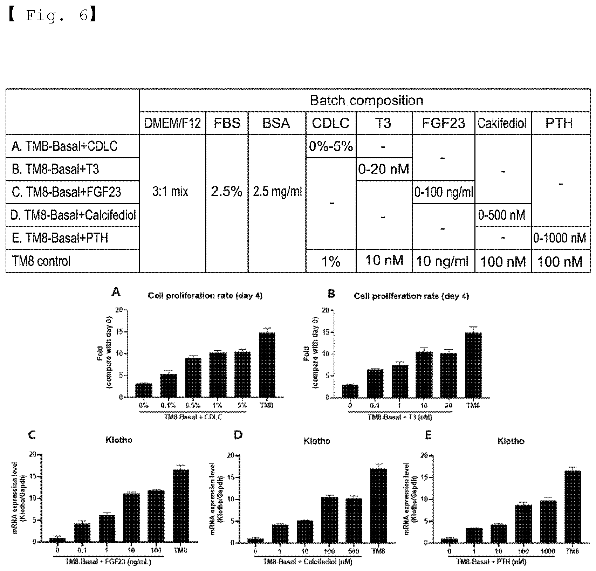

Additionally, in this Example, to explore the optimal concentrations while excluding the interactions among the substances, each substance was individually added to the TM8 basal medium (hereinafter referred to as TM8-Basal), and cell proliferation or klotho gene expression was analyzed and compared. The TM8 medium containing all substances at their optimal concentrations was used as the control group (FIG. 6).

As shown in FIG. 6, it was observed that the addition of each substance to the TM8-Basal resulted in dose-dependent improvements in cell viability or klotho gene expression. The TM8 group containing all substances at their optimal concentrations was found to be the most effective.

Based on the data obtained in this Example, the inventors estimated that the combination of optimal concentrations of the aforementioned additives was the optimal composition of TM8. The performance of TM8 was compared and evaluated against the existing culture media in Table 3 in the previous examples.

EXAMPLE 6 : Klotho Expression Comparison and Analysis

In this Example, the klotho expression characteristics of the derived stem cells were compared and evaluated. Stem cells cultured up to the 5th passage using the media described in Table 3 were analyzed for klotho gene expression and klotho protein expression. Real-time polymerase chain reaction (PCR) analysis was performed for klotho gene expression, and western blotting was conducted using klotho primary antibody (Clone No. KM2076, TransGenic) for klotho protein expression.

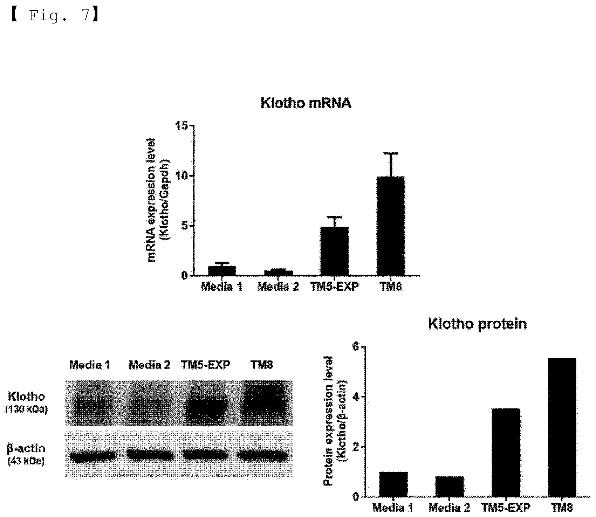

As shown in FIG. 7, it was observed that the derived stem cells cultured with TM8 exhibited the highest level of klotho expression among the media described in Table 3. The klotho expression in TM8 was more than 10-fold higher compared to Media 1 and Media 2, and it was more than 2-fold higher compared to TM5-EXP. The western blotting analysis also confirmed that TM8 had the highest level of klotho protein expression.

These results demonstrate that TM8 significantly enhances klotho expression in derived stem cells compared to Media 1 and Media 2. Moreover, TM8 showed a remarkable improvement in klotho protein expression compared to the previous TM5-EXP medium.

EXAMPLE 7: Inhibition of Renal Fibrosis and Prevention of Podocyte Apoptosis by TM8 and Therapeutic Efficacy of Klotho (in Vitro)

In this Example, the present inventors aimed to evaluate the efficacy of TM8, a medium developed by the present disclosure, in effectively inhibiting renal fibrosis. Initial kidney injury leads to tubular fibrosis, which is considered one of the key triggers for the progression of chronic kidney disease.

To create an in vitro model of tubular fibrosis, human proximal tubular epithelial cells (HK-2) were treated with a representative fibrosis-inducing factor, TGF-$\beta$1, at a concentration of 10 ng/mL for 3 days.

Concurrently, TM8-cultured derived stem cells (UDSCs) from the 5th passage were co-cultured with HK-2 cells for 3 days using a Transwell plate (Falcon, 6-well plate). As a control group, the representative mesenchymal stem cell line adipose-derived stem cells (ADSCs), which do not naturally express klotho, were used. Protein analysis was performed by western blotting to examine proteins involved in the downstream signaling pathway of TGF-$\beta$1.

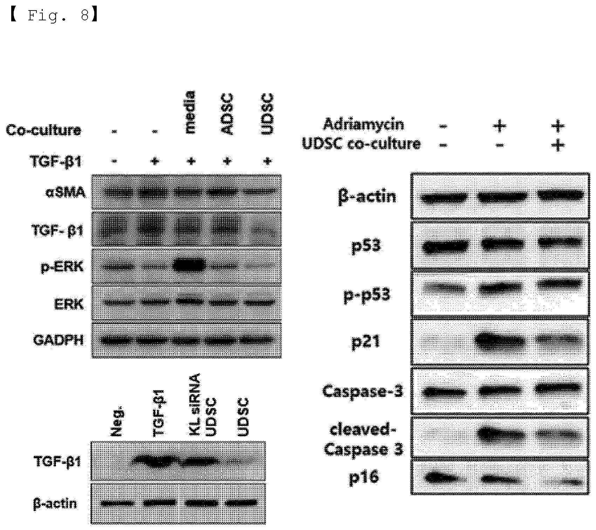

As shown in FIG. 8, TM8-cultured derived stem cells effectively suppressed the expression of TGF-$\beta$1 protein compared to the control group of ADSCs, and the expression of ERK, one of the downstream signaling pathways, was reduced, indicating inhibition of fibrosis progression.

The inventors speculated that the inhibitory effect of TGF-$\beta$1 was attributed to the paracrine effect of Klotho protein secreted by urine-derived stem cells. To investigate this, the effects of urine-derived stem cells cultured in TM8 medium, which enhances klotho protein expression, were compared with the effects of UDSCs in which klotho protein expression was inhibited using siRNA.

As shown in FIG. 8, it was observed that the inhibitory effect of TGF-$\beta$1 was significantly reduced in UDSCs with suppressed klotho protein expression (KL siRNA UDSC). This confirms that the klotho protein secreted by UDSCs plays a crucial role as a therapeutic factor in inhibiting renal fibrosis. Furthermore, it was demonstrated that culturing UDSCs in TM8 medium, which enhances klotho protein expression, improves renal therapeutic function.

Additionally, in this Example, evaluation was made of the ability of UDSCs cultured in TM8 medium to effectively inhibit podocyte apoptosis, which is responsible for the filtration function of the glomerulus. Podocytes play a central role in composing the glomerular basement membrane (GBM) and are known to be crucial for maintaining the glomerular filtration function. It has been observed that chronic kidney diseases and other renal injuries result in podocyte loss due to apoptosis, leading to significant damage to the glomerulus.

To create an in vitro model of podocyte apoptosis, the inventors treated CIHP-1, a human podocyte cell line, with Adriamycin (doxorubicin), a specific toxic drug for podocytes, at a concentration of 0.5 μM for 24 hours to induce fibrosis. Subsequently, UDSCs (urine-derived stem cells) from the 5th passage cultured in TM8 medium were co-cultured with CIHP-1 cells using a Transwell plate (Falcon, 6-well plate) for 3 days. Protein analysis was performed using Western blotting to analyze proteins such as p21, caspase-3, and p16, which are involved in the signaling pathway of p53, a key regulator of the cell cycle.

As shown in FIG. 8, UDSCs cultured in TM8 medium did not directly significantly affect the transcriptional activity of p53. However, the cells were found to inhibit the activation of downstream signals such as p21, caspase-3, and p16. Firstly, the activation of p21, which arrests cell division, was inhibited by UDSCs cultured in the TM8 medium of the present disclosure. Additionally, the activation of caspase-3 into cleaved-caspase-3, which induces apoptosis, was suppressed by UDSCs cultured in the TM8 medium of the present disclosure. Although p16, an indicator of cellular senescence, was not significantly enhanced by Adriamycin treatment in this Example, UDSCs cultured in the TM8 medium of the present disclosure effectively prevented CIHP-1 cells from entering a senescent state. The inventors speculated that this inhibitory effect of p16 is related to the expression of Foxo, an anti-aging factor that was highly expressed in TM5-EXP and TM8 media compared to other comparison media in the gene analysis described in Example 4.

These results demonstrate that UDSCs cultured in TM8 medium have enhanced klotho expression compared to conventional UDSCs, thereby proving their highly effective therapeutic potential in inhibiting renal fibrosis and podocyte apoptosis in kidney diseases such as chronic kidney disease.

EXAMPLE 8: Migration of Urine-Derived Stem Cell into Kidney in Mouse Model of Ischemia Reperfusion Acute Kidney Injury

In this Example, examination was made to determine whether the TM8-cultured UDSCs could migrate into the kidneys of mice with ischemia-reperfusion acute kidney injury, thereby participating in the regeneration process.

To induce ischemic reperfusion acute kidney injury, male BALB/c mice at 8 weeks of age were anesthetized, and the renal artery and vein were observed for 30 minutes. Immediately after inducing renal injury, PKH26-labeled UDSCs from the 4th passage of TM8 culture were administered through the tail vein at a concentration of $1 \times 10^6$ cells/head. After 24 hours of administration, the kidneys were harvested, and the migration of fluorescently labeled stem cells into the damaged kidneys was observed under a fluorescence microscope.

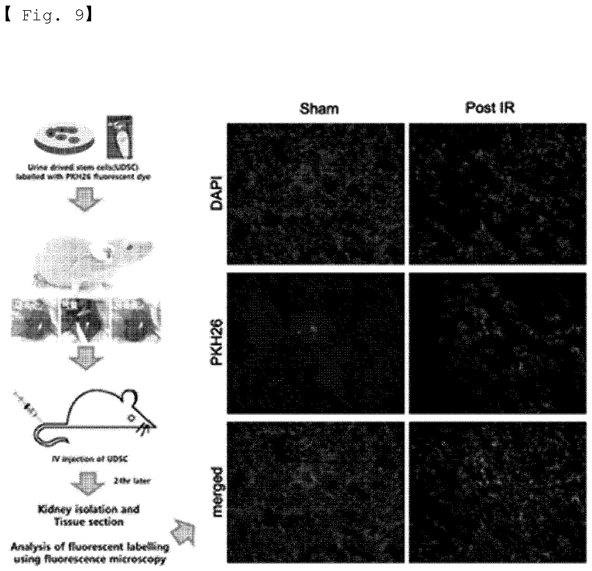

As shown in FIG. 9, the TM8-cultured UDSCs (labeled with PKH26, red) exhibited remarkable migration into the kidneys of mice with ischemic reperfusion acute kidney injury. This observation confirms that the UDSCs derived from TM8 can effectively migrate into the damaged kidneys when administered to mice with ischemic renal injury, thereby demonstrating their involvement in tissue regeneration.

EXAMPLE 9: Efficacy of Urine-Derived Stem Cell in Mouse Model of Ischemia Reperfusion Acute Kidney Injury

In this Example, examination was made to validate the effectiveness of UDSCs derived from TM8 in promoting kidney tissue recovery and inhibiting fibrosis when administered into mice with ischemia-reperfusion acute kidney injury.

To induce ischemia-reperfusion acute kidney injury, mice were subjected to the injury procedure, and after 7 days, UDSCs cultured in TM8 were administered as a single dose at low, medium, and high concentrations (low dose, LS = $4 \times 10^4$ cells/head; medium dose, MS = $2 \times 10^5$ cells/head; high dose, HS = $1 \times 10^6$ cells/head). After 7 days of cell administration, the mice from all experimental groups were sacrificed, and the effectiveness of UDSCs in mitigating kidney injury was evaluated.

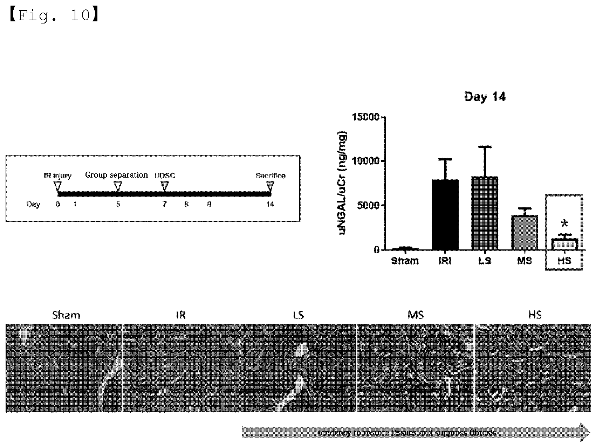

As shown in FIG. 10, when UDSCs cultured in TM8 of the present disclosure were administered at medium and high doses, the levels of NGAL (Neutrophil gelatinase-associated lipocalin), an indicator of acute kidney injury, were significantly reduced compared to the ischemia-reperfusion injury group (IRI). This effect was dose-dependent, with the high-dose (HS) group demonstrating the highest efficacy.

In addition, in this Example, the renal tissue of mice was collected and analyzed for the degree of kidney damage using HE staining.

The results, as shown in FIG. 10, demonstrated a trend of tissue recovery and inhibition of fibrosis in the group treated with urine-derived stem cells cultured in TM8 compared to the ischemia-reperfusion injury group (IRI) . These effects were found to be dose-dependent, with the highest efficacy observed in the high-dose group (HS) .

Therefore, the administration of TM8-cultured urine-derived stem cells exhibited the ability to suppress kidney damage and promote tissue regeneration. The optimal efficacy was observed at the high dose ($1 \times 10^6$/head), as it effectively inhibited tissue damage.

Having described a preferred embodiment of the present invention, it is to be understood that variants and modifications thereof falling within the spirit of the invention may become apparent to those skilled in this art, and the scope of this invention is to be determined by appended claims and their equivalents.

**Claims**

1. A culture medium for isolating and culturing urine-derived stem cells, wherein the culture medium comprises: a basic medium containing Dulbecco's Modified Eagle Medium (DMEM) and Ham's F-12, 5% or less fetal bovine serum, and parathyroid hormone (PTH), and further comprises at least one additive selected from the group consisting of fibroblast growth factor 23 (FGF23), triiodothyronine (T3), calcifediol, bovine serum albumin, and a lipid mixture (chemically defined lipids).

2. The culture medium of claim 1, wherein,

   the basic medium contains Dulbecco's Modified Eagle Medium (DMEM) and Ham's F-12 at a volume ratio of 1:1 to 5:1,
   the fetal bovine serum is contained at 0.1% by volume to 10% by volume, based on the total volume of the medium, and
   the parathyroid hormone (PTH) is contained in an amount of 1 nM to 2,000 nM based on the total concentration of the medium.

3. The culture medium of claim 1 or 2, wherein the basic medium contains Dulbecco's Modified Eagle Medium (DMEM) and Ham's F-12 at a volume ratio of 2:1 to 2:1.

4. The culture medium of claim 1, wherein the fetal bovine serum is contained at 2.5% by volume to 5% by volume, based on the total volume of the medium, and

5. The culture medium of claim 1, wherein the parathyroid hormone (PTH) is contained in an amount of 100 nM to 1,000 nM based on the total concentration of the medium.

6. The culture medium of claim 1, wherein the fibroblast growth factor 23 (FGF23) is contained in an amount of 0.1 ng/mL to 200 ng/mL, based on the volume of the medium.

7. The culture medium of claim 1, wherein triiodothyronine (T3) is contained at a concentration of 0.1 nM to 40 nM, based on the total amount of the medium.

8. The culture medium of claim 1, wherein the calcifediol is contained at a concentration of 1 nM to 1000 nM, based on the total amount of the medium.

9. The culture medium of claim 1, wherein the bovine serum albumin is contained at a concentration of 0.1 mg/mL to 10 mg/mL, based on the total amount of the medium.

10. The culture medium of claim 1, wherein the lipid mixture (chemically defined lipids) is contained at a concentration of 0.1 % by volume to 10 % by volume, based on the total volume of the medium.

11. A method for isolating urine-derived stem cells, the method comprising the steps of:

    (a) sampling urine from a subject;
    (b) centrifuging the urine to collect cells;
    (c) washing the cells, followed by centrifugation; and
    (d) inoculating the cells with the culture medium of claim 1.

12. A method for culturing urine-derived stem cells, the method comprising inoculating the urine-derived stem cells into the culture medium of claim 1.

13. The method of claim 12, wherein the cultured urine-derived stem cells exhibit:

    (1) an increased cell proliferation rate compared to urine-derived stem cells cultured in KSFM and DMEM/F12 (1:1 volume ratio) medium, and

(2) increased expression of the stem cell-related genes OCT4, SOX2, and Nanog, the anti-aging-related genes FOXO1 and FOXO3, and the kidney-specific therapeutic factor klotho compared to urine-derived stem cells cultured in KSFM and DMEM/F12 (1:1 volume ratio) medium.

14. Urine-derived stem cells, cultured in the culture medium of claim 1.

15. The urine-derived stem cells of claim 14, wherein the urine-derived stem cells have an enhanced kidney disease treatment function.

16. The urine-derived stem cells of claim 14, wherein the urine-derived stem cells are positive for CD73, CD90, CD105, CD146, or SSEA-4.

17. The urine-derived stem cells of claim 14, wherein the urine-derived stem cells are autologous, allogeneic, or xenogeneic.

18. The urine-derived stem cells of claim 14, wherein the urine-derived stem cells decrease the neutrophil gelatinase-associated lipocalin (NGAL) level.

19. The urine-derived stem cells of claim 14, wherein the kidney disease is at least one selected from a group consisting of diabetic nephropathy, chronic renal failure, acute renal failure, subacute renal failure, glomerulonephritis, malignant nephrosclerosis, vascular microangiopathy, transplant rejection, glomerulopathy, kidney hypertrophy, kidney proliferation, proteinuria, contrast-induced nephropathy, toxin-induced kidney damage, oxygen free-radical-mediated kidney disease, and nephritis.

20. A cell therapy product with enhanced kidney disease treatment function, wherein the cell therapy product comprises urine-derived stem cells of any one of claims 14 to 19 as an active ingredient.

21. The cell therapy product of claim 20, wherein the cell therapy product may be a preparation adapted to be administered via an oral, rectal, intravenous (i.v), intraarterial, intraperitoneal, intramuscular, intrasternal, transdermal, local, intraocular, subcutaneous, or intradermal route.

22. The cell therapy product of claim 20, further comprising a pharmaceutical carrier.

Fig. 1

Voided urine     Centrifuge     24 well plate colony forming     Well selection     Enzyme treat and expand

UDSC colony

Day 5 (colony forming)

Fig. 2

Fig. 3

|  | Passage | CD31 | CD34 | CD45 | CD73 | CD90 | CD105 | CD146 | SSEA-4 |
|---|---|---|---|---|---|---|---|---|---|
| Media 1 | 3 | 1.30 | 0.01 | 0.65 | 98.72 | 99.57 | 60.99 | 96.04 | 99.87 |
| | 5 | 2.00 | 0.00 | 0.98 | 92.93 | 99.77 | 39.45 | 98.24 | 98.76 |
| Media 2 | 3 | 0.78 | 0.22 | 0.10 | 99.30 | 99.38 | 73.07 | 99.49 | 99.64 |
| | 5 | 2.02 | 0.07 | 0.44 | 93.51 | 99.90 | 57.77 | 99.47 | 99.48 |
| TM5-EXP | 3 | 0.40 | 0.28 | 0.44 | 95.27 | 99.84 | 95.63 | 97.28 | 99.92 |
| | 5 | 0.89 | 0.12 | 0.79 | 99.55 | 99.90 | 93.49 | 96.66 | 99.89 |
| TM8 | 3 | 0.46 | 7.22 | 0.42 | 99.44 | 99.69 | 99.58 | 99.37 | 99.96 |
| | 5 | 0.37 | 2.12 | 0.91 | 99.42 | 99.66 | 99.75 | 99.96 | 99.90 |

|  | Media 1 | | Media 2 | | TM5-EXP | | TM8 | |
|---|---|---|---|---|---|---|---|---|
| Passage | 3 | 5 | 3 | 5 | 3 | 5 | 3 | 5 |
| CD105 | 60.99 | 39.45 | 73.07 | 57.77 | 95.63 | 93.49 | 99.58 | 99.75 |

Fig. 4

Fig. 5

| | Batch composition | | | | | | | |
|---|---|---|---|---|---|---|---|---|
| | DMEM/F12 | FBS | BSA | CDLC | T3 | FGF23 | Cakifediol | PTH |
| A. CDLC test group | 3:1 mix | 2.5% | 2.5 mg/ml | 0%-5% | 10 nM | 10 ng/ml | 100 nM | 100nM |
| B. T3 test group | | | | 1% | 1-20 nM | | | |
| C. FGF23 test group | | | | | 10 nM | 10 ng/ml | | |
| D. Cakifediol test group | | | | | | 10 ng/ml | 0-500 nM | |
| E. PTH test group | | | | | | | 100 nM | 0-1000 nM |

【Fig. 6】

| | Batch composition | | | | | | | |
|---|---|---|---|---|---|---|---|---|
| | DMEM/F12 | FBS | BSA | CDLC | T3 | FGF23 | Cakifediol | PTH |
| A. TMB-Basal+CDLC | | | | 0%-5% | - | | | |
| B. TM8-Basal+T3 | | | | | 0-20 nM | - | - | |
| C. TM8-Basal+FGF23 | 3:1 mix | 2.5% | 2.5 mg/ml | | | 0-100 ng/ml | | - |
| D. TM8-Basal+Calcifediol | | | | - | - | | 0-500 nM | |
| E. TM8-Basal+PTH | | | | | | - | - | 0-1000 nM |
| TM8 control | | | | 1% | 10 nM | 10 ng/ml | 100 nM | 100 nM |

【 Fig. 7】

Klotho mRNA

Klotho protein

【Fig. 8】

【Fig. 9】

【Fig. 10】

## INTERNATIONAL SEARCH REPORT

| International application No. |
|---|
| **PCT/KR2022/005579** |

| **A.** | **CLASSIFICATION OF SUBJECT MATTER** |
|---|---|

**C12N 5/071**(2010.01)i; **A61K 35/22**(2006.01)i; **A61P 13/12**(2006.01)i; **A61K 48/00**(2006.01)i

According to International Patent Classification (IPC) or to both national classification and IPC

| **B.** | **FIELDS SEARCHED** |
|---|---|

Minimum documentation searched (classification system followed by classification symbols)

C12N 5/071(2010.01); A61K 35/12(2006.01); C12N 5/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 요 유래 줄기세포(urine derived stem cell), 분리(separation), 배양(culture), 둘베코 변형 이글 배지(DMEM), 햄의 F-12(Ham's F12), 기본배지(basal medium), 우태아혈청(fetal bovine serum, FBS), 부갑상선호르몬(PTH), 첨가제(additive)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| A | KR 10-2021-0053725 A (EHLBIO CO., LTD.) 12 May 2021 (2021-05-12)<br>See claims 1-3, 9, 11 and 14, paragraphs [0016]-[0019], [0032]-[0035], [0066], [0091], [0099] and [0102], and tables 1-3. | 1-22 |
| A | ANDRUKHOVA, O. et al. Fgf23 and parathyroid hormone signaling interact in kidney and bone. Molecular and Cellular Endocrinology. 2016, vol. 436, pp. 224-239.<br>See entire document. | 1-22 |
| A | WEI, K. et al. Roles of the kidney in the formation, remodeling and repair of bone. Journal of Nephrology. 2016, vol. 29, pp. 349-357.<br>See entire document. | 1-22 |
| A | KR 10-2015-0133516 A (AGENCY FOR DEFENSE) 30 November 2015 (2015-11-30)<br>See entire document. | 1-22 |

| ✓ | Further documents are listed in the continuation of Box C. | ✓ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **04 August 2022** | **04 August 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2022/005579** |

## C.    DOCUMENTS CONSIDERED TO BE RELEVANT

| Category* | Citation of document, with indication, where appropriate, of the relevant passages | Relevant to claim No. |
| --- | --- | --- |
| A | LAVI-MOSHAYOFF, V. et al. PTH increases FGF23 gene expression and mediates the high-FGF23 levels of experimental kidney failure: a bone parathyroid feedback loop. American Journal of Physiology Renal Physiology. 2010, vol. 299, pp. F882-F889.<br>    See entire document. | 1-22 |
| A | TANG, Y. et al. Effects of intermittent parathyroid hormone 1-34 administration on circulating mesenchymal stem cells in postmenopausal osteoporotic women. Medical Science Monitor. 2019, vol. 25, pp. 259-268.<br>    See entire document. | 1-22 |

Form PCT/ISA/210 (second sheet) (July 2019)

**INTERNATIONAL SEARCH REPORT**
**Information on patent family members**

International application No.

**PCT/KR2022/005579**

| Patent document cited in search report | | | Publication date (day/month/year) | Patent family member(s) | | | Publication date (day/month/year) |
|---|---|---|---|---|---|---|---|
| KR | 10-2021-0053725 | A | 12 May 2021 | KR | 10-2320682 | B1 | 03 November 2021 |
| KR | 10-2015-0133516 | A | 30 November 2015 | KR | 10-1636405 | B1 | 05 July 2016 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020210053725 A

- KR 1020190139702

**Non-patent literature cited in the description**

- Remington's Pharmaceutical Sciences. Mack Publishing Company, 1995